# EUROPEAN PATENT APPLICATION

(11) **EP 3 144 027 A1**
(43) Date of publication of application: **22.03.2017**
(21) Application number: 15185643.2
(22) Date of filing: 17.09.2015
(51) Int. Cl.: A61M 25/01, A61M 16/04

(54) **PROTECTIVE SLEEVE FOR SUCTION CATHETERS**

(71) Applicant: Vitaltec Corporation, Taichung City (TW)
(72) Inventor: CHIU, Sheng-Yu, Taichung City (TW); TSAI, Hsien-Chih, Taichung City (TW)
(74) Representative: Pallini Gervasi, Diego

(57) **Abstract**

A protective sleeve (10, 10A, 10B) has at least one protective slice (20, 20A, 20B, 40, 40A) and at least one limiting string (30, 30A, 30B). The at least one protective slice (20, 20A, 20B, 40, 40A) is an elongated membrane slice and has two sticking portions (21, 21A, 21B, 41, 41A) formed on the same surface of the at least one protective slice (20, 20A, 20B, 40, 40A). The at least one limiting string (30, 30A, 30B) is each a low-extended string and embedded in one of the sticking portions (21, 21A, 21B, 41, 41A) of the at least one protective slice (20, 20A, 20B, 40, 40A). When the protective sleeve (10, 10A, 10B) is pulled, the extension length of the protective sleeve (10, 10A, 10B) is limited. The manufacturing procedure is simplified, which can reduce the manufacturing cost.

## Description

### 1. Field of the Invention

The present invention relates to a protective device for suction catheters, and more particularly to a protective sleeve for suction catheters that can simplify the manufacturing procedure and reduce the manufacturing cost.

### 2. Description of Related Art

Some patients cannot breathe by themselves after a surgery, so a ventilation machine is needed to supply oxygen for the patients. However, the secretions and the phlegm may block the trachea of the patients, so a suction catheter is used to draw the secretions and the phlegm from the trachea. A conventional suction catheter comprises a connecting base, a catheter, a control valve, and a protective sleeve. The connecting base is connected to a vent tube which communicates with a patient's trachea and has a positioning segment formed in the connecting base. The catheter has a proximal end and a distal end. The proximal end of the catheter is mounted through and abuts the positioning segment of the connecting base securely, and the proximal end of the catheter is inserted in the connecting base. The control valve is connected to the distal end of the catheter.

The protective sleeve is hollow, covers around the catheter, and is made of extendible plastic. The protective sleeve is connected to the connecting base and the control valve to mount around the catheter to hold the catheter in a closed condition. So the catheter is not exposed to the air directly, and this can prevent the secretions and the phlegm from exposure in the air. Therefore, an interior of the connecting base does not communicate with an interior of the protective sleeve because the proximal end of the catheter is mounted through and abuts the positioning segment of the connecting base securely. The protective sleeve only covers around an outer surface of the connecting base. Then, the oxygen from the ventilation machine does not flow in the interior of the protective sleeve via the connecting base.

When the conventional suction catheter is in use, the proximal end of the catheter is inserted into the trachea, and then presses the control valve to draw the secretions and the phlegm from a patient. One hand of the user holds the protective sleeve and withdraws the proximal end of the catheter back to the connecting base. The catcher can draw the secretions and the phlegm continually, and this can make the patient not so uncomfortable. However, the protective sleeve is made of extendible plastic and is elastic and extendible, and once the user over-withdraws the catheter, this makes the proximal end of the catheter inserted into the interior of the protective sleeve via the positioning segment of the connecting base. So the connecting base would communicate with the interior of the protective sleeve. The oxygen of the ventilation machine would flow into the interior of the protective sleeve via the connecting base, such that the protective sleeve expands and may be damaged and exploded, and this makes the secretions and the phlegm exposed in the air directly.

US Patent No. 8,567,401 is provided and entitled "Looped tether for medical ventilating and aspirating devices". In the US Patent, an appropriate length of cord is cut and two ends of the cord are tied together with a knot. On a proximal end of the catheter, the looped tether may be wrapped around a proximal end fitting and slipped under the plurality of knot stays. On a distal end of the catheter, the loop of the looped tether is hooked over at least one loop tab formed in a cleft on the proximal end of a distal fitting. The looped tether may limit the extension length of the sleeve. So the looped tether can prevent the over-withdrawal of a suction catheter from a connecting fitting in a closed suction catheter respiratory device. However, the manufacturing process of the looped tether wrapped around the proximal fitting and hooked over at least one loop tab may be complicated and increase the manufacturing cost.

To overcome the shortcomings of the conventional protective sleeve for suction catheters, the present invention provides a protective sleeve for suction catheters to mitigate or obviate the aforementioned problems.

The main objective of the present invention is to provide a protective sleeve for suction catheters which could simplify the manufacturing procedure and reduce the manufacturing cost.

The protective sleeve has at least one protective slice and at least one limiting string. Each one of the at least one protective slice is a rectangular membrane slice and has two sticking portions formed on the same surface of the at least one protective slice. The at least one limiting string is a low-extended string and embedded in one of the sticking portions of the at least one protective slice. When the protective sleeve is pulled, the extension length of the protective sleeve is limited. The manufacturing procedure is simplified, which can reduce the manufacturing cost.

Other objects, advantages, and novel features of the invention will become more apparent from the following detailed description when taken in conjunction with the accompanying drawing.

### IN THE DRAWINGS:

Fig. 1 is a perspective view of a first embodiment of a protective sleeve for suction catheters in accordance with the present invention;
Fig. 2 is an exploded perspective view of the protective sleeve in Fig. 1;
Fig. 3A is an enlarged and cross sectional side view of the protective sleeve in Fig. 1;
Fig. 3B is an enlarged and cross sectional side view of the protective sleeve in Fig. 3A;
Fig. 4 is an operational perspective view of the protective sleeve in Fig. 1;
Fig. 5 is another operational perspective view of the protective sleeve in Fig. 1;
Fig. 6A is an enlarged and cross sectional side view of a second embodiment of the protective sleeve in accordance with the present invention;
Fig. 6B is an enlarged and cross sectional side view of the protective sleeve in Fig. 6A;
Fig. 7 is an exploded perspective view of a third embodiment of the protective sleeve in accordance with the present invention;
Fig. 8A is an enlarged and cross sectional side view of the protective sleeve in Fig. 7; and
Fig. 8B is an enlarged and cross sectional side view of the protective sleeve in Fig. 8A.

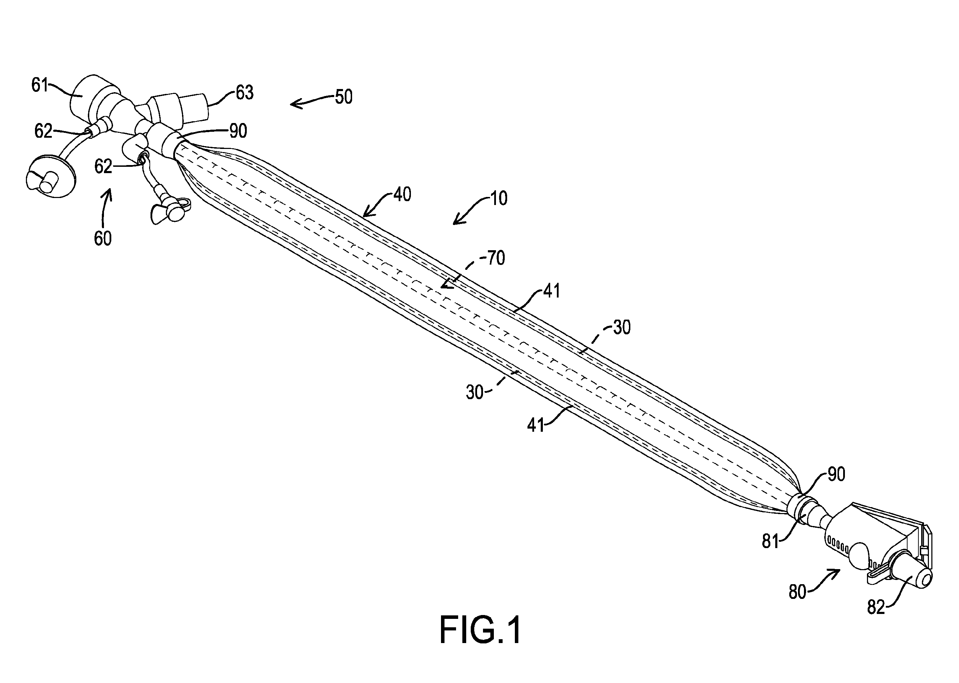

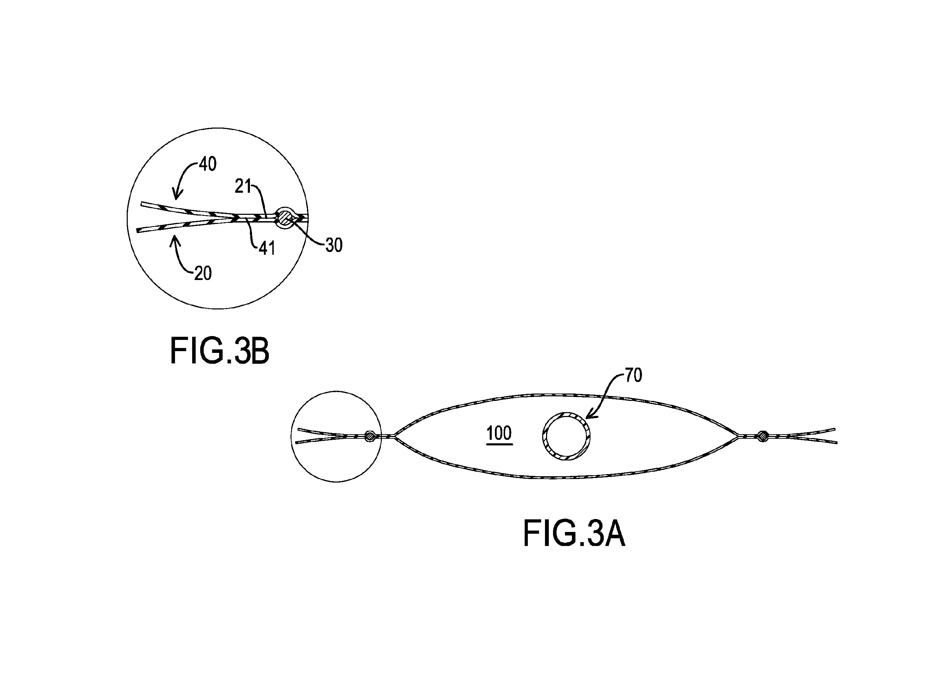

With reference to Figs. 1 to 3A, a first embodiment of a protective sleeve 10 for suction catheters in accordance with the present invention is shown. The protective sleeve 10 is connected to a transmitting assembly 50.

The transmitting assembly 50 comprises a connecting base 60, a catheter 70, a control valve 80, and two collars 90. The connecting base 60 is a cylindrical tube and has a surrounding wall, two open edges, a joining port 61, at least one access port 62, a vent port 63, a connecting port 64, and a positioning segment. Preferably, the transmitting assembly 50 comprises two access ports 62. The joining port 61 and the connecting port 64 are formed on the two open edges of the connecting base 60 respectively. The two access ports 62 and the vent port 63 are formed on the surrounding wall of the connecting base 60. The positioning segment is formed in the connecting base 60 and is integrated with the connecting port 64, and this makes the joining port 61 communicate with the two access ports 62, the vent port 63, and the connecting port 64. The connecting port 64 has an external surface.

The catheter 70 is mounted through the connecting base 60 and has an external surface, a proximal end 71 and a distal end 72. The proximal end 71 is mounted through the connecting base 60 via the connecting port 64 and the positioning segment, and this makes the external surface of the catheter 70 abut the positioning segment of the connecting base 60. The control valve 80 is mounted around the catheter 70 and has two open edges, a fixing port 81, and a pump port 82. The fixing port 81 and the pump port 82 are formed on the two open edges of the control valve 80 respectively and aligned with each other. The distal end 72 of the catheter 70 is mounted trough the control valve 80. One of the two collars 90 is mounted around the protective sleeve 10 and then engages the connecting port 64 of the connecting base 60, and the other collar 90 is mounted around the protective sleeve 10 and then engages the fixing port 81 of the control valve 80. Then, the protective sleeve 10 is positioned between the connecting base 60 and the control valve 80.

The protective sleeve 10 is tube-shaped, covers around the catheter 70 and has two open edges. The two open edges of the protective sleeve 10 engage the connecting base 60 and the control valve 80 via the two collars 90 respectively. The protective sleeve 10 has at least one protective slice and at least one limiting string.

With reference to Figs. 1 to 3B, the protective sleeve 10 of the first embodiment of the present invention has a lower protective slice 20, two limiting strings 30, and an upper protective slice 40.

The lower protective slice 20 is a rectangular membrane slice and has a lower surface, an upper surface, two long sides, two short sides, and two sticking portions 21. The two sticking portions 21 are formed on the upper surface of the lower protective slice 20 and are adjacent to the two long sides of the lower protective slice 20 respectively. The two sticking portions 21 are disposed at an interval, and are respectively parallel to the two long sides of the lower protective slice 20. The two short sides of the lower protective slice 20 are respectively defined as a proximal end 201 and a distal end 202. The two limiting strings 30 are low-extended strings. Preferably, the two limiting strings 30 are cotton strings. The two limiting strings 30 are respectively stuck on the two sticking portions 21 along the two long sides of the lower protective slice 20.

The upper protective slice 40 corresponds in shape to the lower protective slice 20 and has an upper surface, two long sides, a lower surface, and two sticking portions 41. The two sticking portions 41 are formed on the lower surface of the upper protective slice 40 and are adjacent to the two long sides of the upper protective slice 40 respectively. The two sticking portions 41 are disposed at an interval, and are parallel to the two long sides of the upper protective slice 40. The two short sides of the upper protective slice 40 are respectively defined as a proximal end 401 and a distal end 402. The two sticking portions 41 of the upper protective slice 40 are stuck on the two sticking portions 21 of the lower protective slice 20 respectively, such that the two limiting strings 30 are embedded between the two sticking portions 21 of the lower protective slice 20 and the two sticking portions 41 of the upper protective slice 40 respectively. A receiving space 100 is defined between the lower protective slice 20, the upper protective slice 40, and the two limiting strings 30. The extension length of the protective sleeve 10 is limited when the two protective slices 20,40 are pulled in a direction which is along the two long sides of the protective slices 20, 40, because the two limiting strings 30 cannot be extended. Preferably, the two protective slices 20, 40 are stuck to each other by high frequency pressing method.

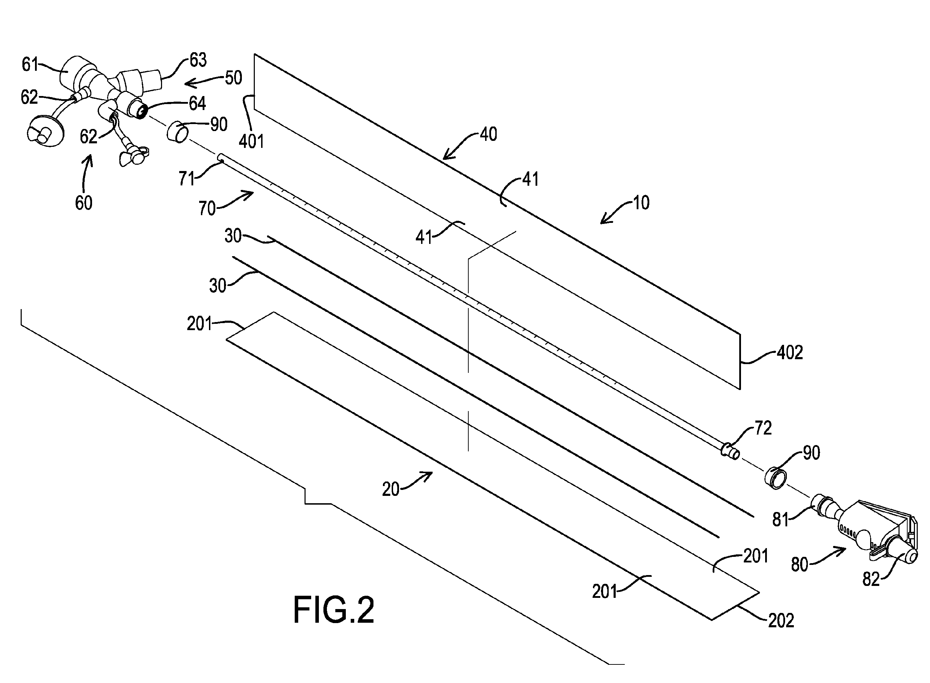

With reference to Figs. 1 and 2, the protective sleeve 10 of the first embodiment is connected to the transmitting assembly 50 to form a suction catheter. The proximal end 71 of the catheter 70 is located in the connecting base 60 via the connecting port 64 and the positioning segment, and the distal end 72 of the catheter 70 is mouthed through and engages the control valve 80. The two collars 90 are mounted around a middle of the protective sleeve 10, and the proximal end 201 of the upper surface of the lower protective slice 20 and the proximal end 401 of the lower surface of the upper protective slice 40 are attached to the external surface of the connecting port 64 of the connecting base 60. The upper surface of the lower protective slice 20 adjacent to the distal end 202 is attached to the external surface of the fixing port 81 of the control valve 80.The upper surface of the upper protective slice 40 adjacent to the distal end 402 is attached to the external surface of the fixing port 81 of the control valve 80.

Then, the two collars 90 are pushed toward the two open edges of the protective sleeve 10 respectively. One of the two collars 90 is mounted around and fixed on the proximal end 201 of the lower protective slice 20, the proximal end 401 of the upper protective slice 40, and the connecting port 64 of the connecting base 60. The other collar 90 is mounted around and fixed on the distal end 202 of the lower protective slice 20, the distal end 402 of the upper protective slice 40, and the fixing port 81 of the control valve 80. An interior of the connecting base 60 does not communicate with the receiving space 100 because the external surface of the proximal end 71 of the catheter 70 abuts the positioning segment of the connecting base 60 securely. The two open edges of the protective sleeve 10 are fixed on the connecting base 60 and the control valve 80 respectively, and the protective sleeve 10 covers around the catheter 70 to form a closed condition, so the catheter 70 is not exposed to the air directly.

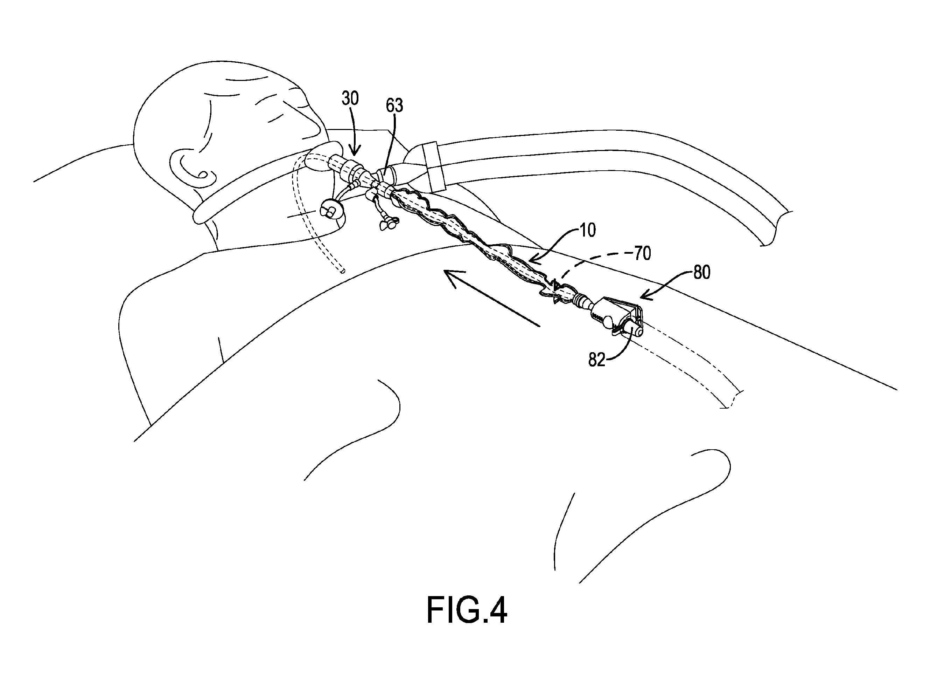

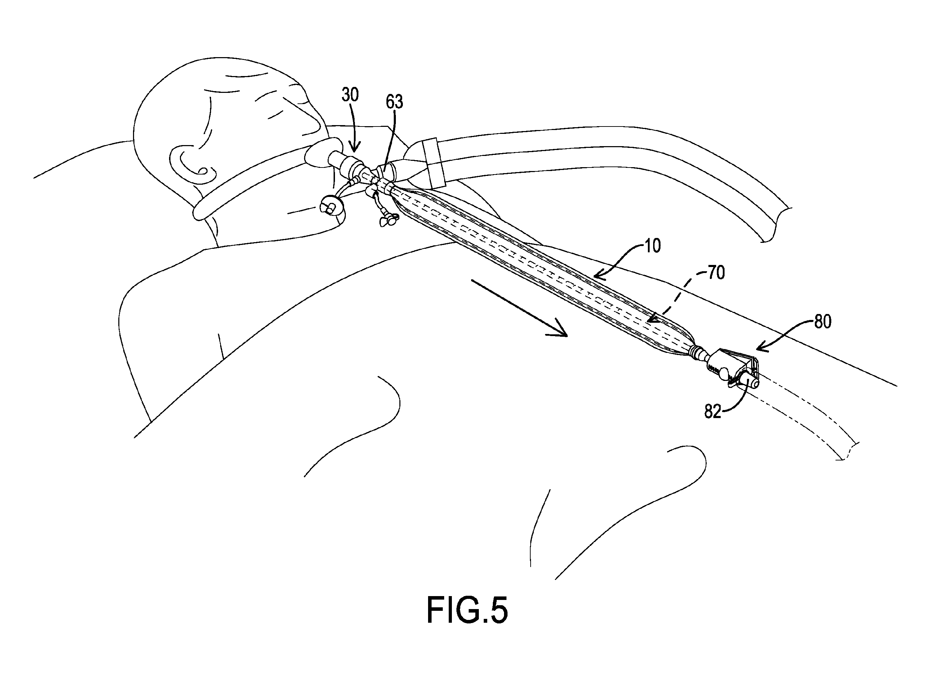

With the reference to the Figs. 1, 4, and 5, the suction catheter that is formed by the protective sleeve 10 and the transmitting assembly 50 has the following operation procedures.
1. The joining port 61 of the connecting base 60 is connected to a vent tube which communicates with a patient's trachea. A valve portion is formed in the vent tube. The vent port 63 of the connecting base 60 is connected to a ventilation machine, and the ventilation machine can supply air to the patient continually via the joining port 61. In addition, the pump port 82 of the control valve 80 is connected to a vacuum device.
2. One hand of a user holds the connecting base 60, and the other hand of the user holds the protective sleeve 10, and then moves the catheter 70 into a proper depth in the patient's trachea.
3. By pressing the control valve 80, the vacuum device can force a suction force to the catheter 70, and the secretions and the phlegm may be removed by the catheter 70. The hand which holds the protective sleeve 10 keeps holding the protective sleeve 10, and then withdraws the catheter 70 slowly, and this makes the proximal end 71 of the catheter 70 move back into the interior of the connecting base 60. During the operating procedure, the extended length of the protective sleeve 10 in the direction which is along the two long sides of the protective slices 20, 40 is limited, because the two limiting strings 30 are embedded between the two protective slices 20, 40. Therefore, the proximal end 71 of the catheter 70 would not move to the receiving space 100 because the extended length of the protective sleeve 10 in the direction which is along the two long sides of the protective slices 20, 40 is limited, and this can prevent the air in the ventilation machine from entering the receiving space 100 via the connecting port 64 and the positioning segment of the connecting base 60. So the two protective slices 20, 40 would not be broken or exploded.
4. After the proximal end 71 of the catheter 70 moves back to the interior of the connecting base 60, saline solution or medication flows in the connecting base 60 via the access port 62 when the secretions and the phlegm are too thick. The patient's secretions and phlegm become thinner, such that the catheter 70 can withdraw the patient's secretions and phlegm from the connecting base 60. Additionally, the valve portion of the vent tube can prevent the secretions and the phlegm from flowing back to the patient via the joining port 61 of the connecting base 60.

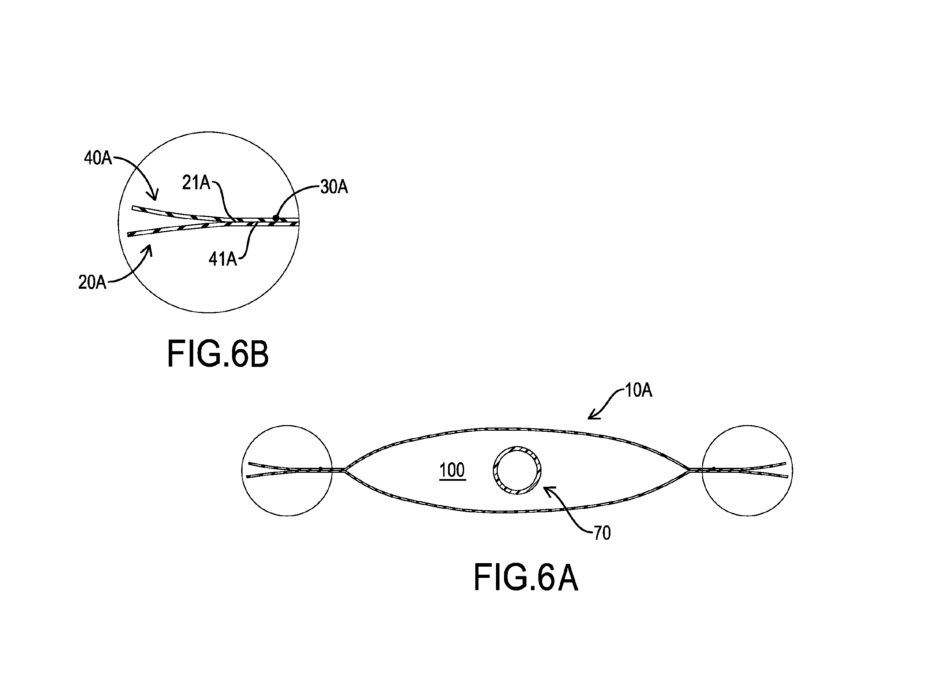

With reference to Figs. 6A and 6B, a second embodiment of a protective sleeve for suction catheters in accordance with the present invention is similar to the first embodiment, and the differences are: when the two protective slices 20A, 40A are stuck to each other by high frequency pressing, the two limiting strings 30A are embedded in the upper surface of the upper protective slice 40A and above the two sticking portions 41A respectively. The combination method and operation procedure of the protective sleeve 10A and the transmitting assembly 50 of the second embodiment is the same as the first embodiment, and detailed description thereof will be omitted.

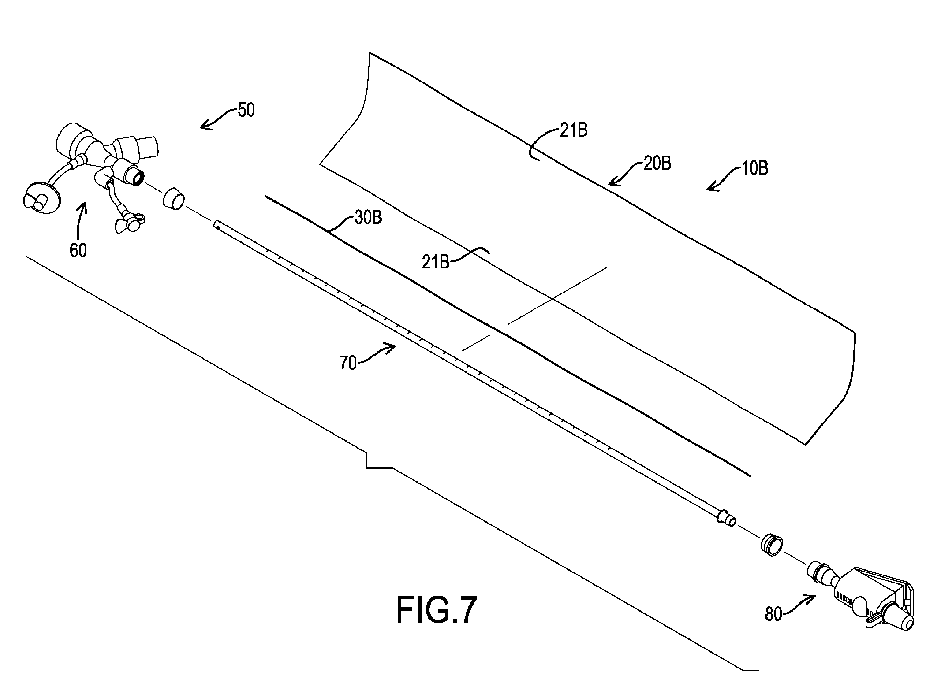

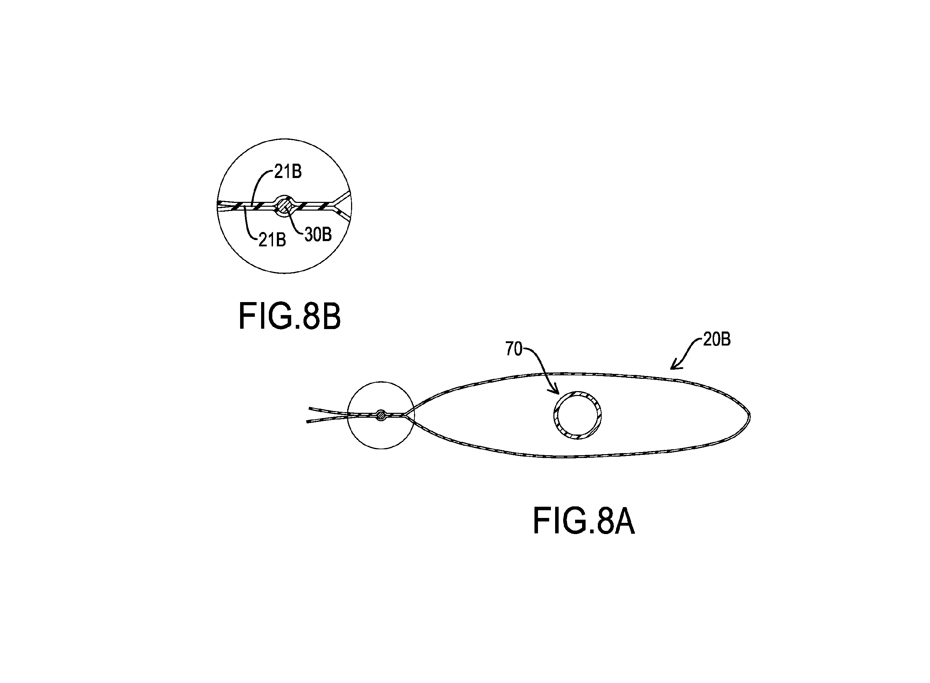

With reference to Figs. 7, 8A, and 8B, a third embodiment of a protective sleeve for suction catheters in accordance with the present invention is similar to the first embodiment, and the differences are: the protective sleeve 10B has a protective slice 20B and a limiting string 30B, the protective slice 20B is a folded rectangular membrane slice and has an internal surface, two long sides, and two sticking portions 21B. The two sticking portions 21B are formed on the internal surface of the protective slice 20B and are parallel to the two long sides of the protective slice 20B. The two sticking portions 21B are stuck on and face to each other because the protective slice 20B is folded. The limiting string 30B is embedded between the two sticking portions 21B respectively.

The protective sleeve for suction catheters in accordance with the present invention as described has the following advantage. The at least one limiting string 30, 30A, 30B is embedded in the at least one protective slice 20, 20A, 20B, 40, 40A directly. The at least one limiting string 30, 30A, 30B is disposed along the direction which is along the two long sides of each protective slice 20, 20A, 20B, 40, 40A. So the extension length of the protective sleeve 10, 10A, 10B is limited when the protective sleeve 10, 10A, 10B is pulled. Compared with the conventional protective sleeve for suction catheters, the loop of the looped tether is hooked over at least one loop tab formed in a cleft on the proximal end of a distal fitting. Therefore, the manufacturing process of the looped tether wrapped around a proximal fitting and hooked over at least one loop tab may be complicated and increase the manufacturing cost. The manufacturing procedure of the present invention is simplified, which can reduce the manufacturing cost.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A protective sleeve for suction catheters, **characterized in that** the protective sleeve comprises:
at least one protective slice (20, 20A, 20B, 40, 40A) each being an elongated slice and having
two long sides;
two surfaces; and
two sticking portions (21, 21A, 21B, 41, 41A) formed on the same surface of the at least one protective slice (20, 20A, 20B, 40, 40A) and adjacent to the two long sides of the at least one protective slice (20, 20A, 20B, 40, 40A) respectively; and
at least one limiting string (30, 30A, 30B) each being a low-extended string and embedded in one of the sticking portions (21, 21A, 21B, 41, 41A) of the at least one protective slice (20, 20A, 20B, 40, 40A).

2. The protective sleeve for suction catheters as claimed in claim 1, wherein one protective slice (20B) and one limiting string (30B) are implemented; wherein
the protective slice (20B) is a folded elongated slice, and the two sticking portions (21B) are stuck on and face to each other; and
the limiting string (30B) is embedded between the two sticking portions (21B).

3. The protective sleeve for suction catheters as claimed in claim 1, wherein two protective slices (20, 40) and two limiting strings (30) are implemented; wherein
the two protective slices (20, 40) are a lower protective slice (20) and an upper protective slice (40); wherein
the lower protective slice (20) has
an upper surface; and
two sticking portions (21) disposed at an interval on the upper surface of the lower protective slice (20);
the two limiting strings (30) are stuck on the two sticking portions (21) of the lower protective slice (30) respectively; and
the upper protective slice (40) has
a lower surface; and
two sticking portions (41) disposed at an interval on the lower surface of the upper protective slice (40) and facing the two sticking portions (21) of the lower protective slice (20) respectively, wherein the two limiting strings (30) are embedded between the corresponding sticking portions (21, 41) of the two protective slices (20, 40).

4. The protective sleeve for suction catheters as claimed in claim 1, wherein two protective slices(20A, 40A) and two limiting strings (30A) are implemented; wherein
the two protective slices (20A, 40A) are a lower protective slice (20A) and a upper protective slice (40A); wherein
the lower protective slice (20A) has
an upper surface; and
two sticking portions (21A) disposed at an interval on the upper surface of the lower protective slice (20A);
the upper protective slice (40A) has
an upper surface; and
two sticking portions (41A) disposed at an interval and facing the two sticking portions (21A) of the lower protective slice (20A) respectively; and
the two limiting strings (30A) are embedded in the upper surface of the upper protective slice (40A) and above the two sticking portions (41A) respectively.

5. The protective sleeve for suction catheters as claimed in any one of claims 1 to 4, wherein the two sticking portions (21, 21A, 21B, 41, 41A) are stuck on each other by high frequency pressing.

6. The protective sleeve for suction catheters as claimed in claim 5, wherein the two sticking portions (21, 21A, 21B, 41, 41A) are parallel to each other.

7. The protective sleeve for suction catheters as claimed in claim 6, wherein each limiting string (30, 30A, 30B) is a cotton string.
